# EUROPEAN PATENT SPECIFICATION

(11) **EP 2 427 126 B1**
(45) Date of publication and mention of the grant of the patent: **19.02.2020**
(21) Application number: 10721316.7
(22) Date of filing: 10.05.2010
(51) Int. Cl.: A61B 17/06

(54) **COATED THREAD WITH ANCHORING STRUCTURES FOR ANCHORING IN BIOLOGICAL TISSUES**
BESCHICHTETER FADEN MIT VERANKERUNGSMERKMALEN ZUR VERANKERUNG IN BIOLOGISCHEN GEWEBEN
FILS REVÊTUS PRÉSENTANT DES STRUCTURES D'ANCRAGE POUR ANCRAGE DANS DES TISSUS BIOLOGIQUES

(30) Priority: 08.05.2009 DE 102009020901
(43) Date of publication of application: 14.03.2012
(73) Proprietor: Aesculap AG, 78532 Tuttlingen/Donau (DE)
(72) Inventor: ODERMATT, Erich, 8200 Schaffhausen (CH); BERNDT, lngo, 78532 Tuttlingen (DE); KÖNIG, Silke, 78628 Rottweil (DE); OBERHOFFNER, Sven, 71384 Weinstadt-Endersbach (DE); MÜLLER, Erhard, 70565 Stuttgart (DE)
(74) Representative: Patentanwälte Ruff, Wilhelm, Beier, Dauster & Partner mbB
(86) International application number: PCT/EP2010/002847
(87) International publication number: WO 2010/127874

(56) References cited:
- WO-A2-2009/042841
- US-A- 5 222 976
- US-A1- 2005 171 588
- US-A1- 2005 267 532
- US-B1- 6 264 675

## Description

The present invention relates to a thread that is a knotless or self-fixing surgical suture material, to a surgical implant, to a surgical kit and to a method for producing the thread.

Thread-like suture materials are used as standard in surgery for closure of wounds. They are usually knotted in order to obtain a secure fixing in the tissue. Care has to be taken to ensure that the wounds to be closed are sutured with an optimal force at the wound margins. If the wound margins are sutured too loosely and too irregularly, for example, there is in principle a risk of increased scar formation or dehiscence. By contrast, if the wound margins are sutured too strongly, there is a danger of the circulation of blood in the wound margins being restricted, which can result in necrotic changes in the surrounding tissue area.

In addition to the risk of possible complications, in particular further surgical interventions, there is therefore always a degree of risk of the wound repair, based on knotting of suture materials, leading to impaired healing and to unsatisfactory cosmetic results in the patients concerned. Another consideration is that several knots often have to overlap in order to achieve a secure knot hold. This entails introducing a large amount of material into the area of the wound that is to be treated and can lead to increased foreign-body reactions, particularly in the case of resorbable suture materials.

Suture materials which, in contrast to conventional threads, do not have to be knotted have long been known under the term "barbed sutures". Such knotless or self-fixing suture materials are usually composed of a monofilament thread which, along its longitudinal axis, has structures called barbs. Corresponding suture materials are described, for example, in documents US 3,123,077 A, EP 1 559 266 B1, EP 1 560 683 B1 and EP 1 556 946 B1. The barbs are usually formed on a thread in such a way that the thread can be pulled through a tissue along the direction of the barbs. When a pull is exerted in the opposite direction, the barbs can stand upright and anchor themselves, and therefore also the thread, in the surrounding tissue area. This ensures that the thread cannot be pulled back through the incision channel.

Although the thread, as mentioned in the preceding paragraph, is generally pulled through a biological tissue along the direction of its barbs, tissue trauma caused by the barbs can never be entirely ruled out. To overcome this problem, the use of a tubular insertion device is proposed in US 6,241,747 B1, US 5,342,376 and DE 10 2005 004 318 A1. The insertion device in the first instance avoids direct contact of the barbs with the body tissue. It is only after the suture material has been correctly positioned that the insertion device is removed, exposing the barbs, and the barbs are able to anchor themselves in the surrounding tissue area. The use of insertion devices, however, makes surgical techniques employing them complicated and susceptible to error.

US 6,264,675 B1 discloses a self-anchoring suture having anchoring structures in the form of barb members which are partially coated by an adhesive.

WO 2009/042841 A2 discloses a self-retaining suture having retainers which are partially coated by a supplementary material.

US 2005/0267532 A1 discloses a surgical thread for plastic surgery operations comprising multiple sharp projections.

US 2005/0171588 A1 discloses a lead which may be coupled to a therapy generator and which may be inserted through blood vessels.

The object of the present invention is therefore to make available a knotless or self-fixing suture material that can be pulled through tissue with the least possible trauma, and without the need to use additional aids.

This object is achieved by a thread having the features of independent claim 1. Preferred embodiments of the thread according to the invention are the subject matter of dependent claims 2 to 13. The invention further relates to a surgical implant according to claim 14. A further aspect of the invention concerns a surgical kit having the features of claim 15. Likewise, the present invention relates to a method for producing the thread, in accordance with claim 16. The wording of all the claims is herewith incorporated by reference into the content of this description.

The thread according to the invention is a thread comprising a main body, and anchoring structures that are used for anchoring in biological tissues, in particular human and/or animal tissues, and that are formed on the surface of the main body of the thread. The main body of the thread is generally designed as an elongate body. At least some of the anchoring structures are covered at least partially, preferably completely, by a coating of the main body of the thread, which coating can be removed by means of liquids, in particular body fluids. Preferably, all the anchoring structures are covered by the coating of the main body of the thread. The thread according to the invention is a knotless or self-fixing surgical suture material.

Biocompatible liquids, or preferably body fluids themselves, are preferable as the liquids for removing the coating provided according to the invention. Examples of suitable biocompatible liquids are chosen from the group consisting of water, salt solutions, electrolyte solutions, buffer solutions and sugar solutions. Body fluids are to be understood in particular, within the meaning of the present invention, as tissue fluid, blood, lymph, wound fluid or exudate.

The biological tissues within the meaning of the present invention can be, for example, skin, fat, fascias, bones, muscles, organs, nerves, blood vessels, connective tissues, tendons or ligaments.

At least some of the anchoring structures are completely embedded in the coating.

The anchoring structures are fixed, by the coating, in a certain position on the surface of the main body of the thread. Preferably, at least some of the anchoring structures are fixed, by the coating, in a position protruding from the surface of the main body of the thread. Particularly preferably, all the anchoring structures are fixed, by the coating, in a position protruding from the surface of the main body of the thread. In particular, the anchoring structures may be pressed onto the surface of the main body of the thread.

In an alternative embodiment, at least some of the anchoring structures are fixed, by the coating, in a position bearing on the surface of the main body of the thread. In other words, at least some of the anchoring structures in this embodiment do not protrude from the surface of the main body of the thread. According to the invention, it is particularly preferable if all the anchoring structures are fixed, by the coating, in a position bearing on the surface of the main body of the thread.

The coating can preferably dissolve in liquids or upon contact with liquids or is soluble in such liquids. The coating can preferably dissolve in or upon contact with body fluids or is soluble in body fluids.

This advantageously avoids the need for measures for removing the coating, which measures would otherwise have to be taken by a user, for example by a surgeon. It is particularly advantageous if the coating dissolves in or upon contact with body fluids only after a certain period of time. In this way, the user of the thread according to the invention is given sufficient time to carry out a repositioning of the thread if this proves necessary. For example, the coating can be designed such that it dissolves in or upon contact with body fluids only after a few minutes. In other words, it is particularly preferable, according to the invention, if the coating is made from a material that dissolves in or upon contact with body fluids but after some delay.

However, it is also possible in principle, according to the invention, for the coating to dissolve in or upon contact with biocompatible liquids or to be soluble in such liquids. In this way, after correct positioning of the thread, the coating can be removed, for example, by a simple flushing procedure. According to the invention, it is also possible for the embodiments described in the preceding paragraph to be combined with a flushing procedure in order, if appropriate, to accelerate the removal of the coating.

After removal of the coating, the anchoring structures preferably protrude from the main body of the thread.

The coating itself is preferably formed in the manner of a sheath on the surface of the main body of the thread. The coating preferably completely surrounds the main body of the thread, including the anchoring structures. The coating is generally formed with a uniform layer thickness on the surface of the main body of the thread. The coating preferably has a radius component of between 5 and 100%, in particular 10 and 50%, relative to a radius of the main body of the thread (without anchoring structures protruding therefrom). In principle, the thread can have a coating that amounts to between 3 and 70% by weight relative to the total weight of the thread. Small amounts of coating are preferred, since the removal of the coating can be accelerated in this way. By means of the coating, the thread preferably has a friction-reducing outer surface, preferably a substantially flat outer surface.

Moreover, the coating can be configured as a film, sleeve, sheath, encasing, membrane, sponge, foam or gel, in particular hydrogel, on the surface of the main body of the thread.

In a preferred embodiment, the coating is configured as a tube or hose. More preferably, the tube or hose comprises anchoring structures, in particular in the sense of the present invention. In principle, the anchoring structures may protrude into the interior and/or exterior of the tube or hose. Preferably, the anchoring structures protrude into the exterior of the tube or hose. Further, the anchoring structures may be configured as cuts into the surface of the main body, wherein the cuts preferably do not break through the wall of the tube or hose. As an alternative or in combination, the anchoring structures may be configured as breakthroughs, i.e. the anchoring structures are formed completely breaking through the wall of the tube or hose. A coating that is configured as a tube or hose having anchoring structures is especially advantageous, since it imparts the thread self-anchoring properties even immediately upon implantation of the thread into the body of a patient. A tubular or hose-like coating may be applied on the thread by means of shrinkage and/or drawing. In general a tubular or hose-like coating having anchoring structures as described herein, may be applied, in particular shrunk and/or drawn, on a round stock, fibre, monofilament, pseudomonofilament, multifilament, thread, yarn or the like so as to equip these structures with self-anchoring properties. In order to accelerate the dissolution of the coating by means of liquids, in particular body liquids and/or biocompatible liquids, the coating may have a small wall thickness.

In another embodiment, the coating is porous, in particular with open pores. The coating preferably has a porosity of between 30 and 90%, in particular 60 and 80%, relative to the total volume of the coating. The higher the porosity of the coating, the less coating material has to be removed in order to expose the anchoring structures on the surface of the main body of the thread.

In another embodiment, the main body of the thread and the anchoring structures are formed integrally. The anchoring structures are preferably formed as cuts in the surface of the main body of the thread. As has already been mentioned, the anchoring structures can protrude from the surface of the main body of the thread. The cuts can be mechanical cuts, physicochemical cuts, in particular laser-generated cuts, or thermal cuts. To form mechanical cuts, cutting blades can be used for example. Suitable cutting blades are often part of a cutting device, which additionally comprises a cutting board (cutting abutment) and also holding or fixing elements, for example a vice, chucks, holding or clamping jaws, for the thread that is to be cut. Thermal cuts can be generated, for example, by means of a heated cutting blade or a heated cutting wire, in particular an electrically heated cutting wire. To form laser-generated cuts, it is possible in principle to use gas lasers, for example CO₂ lasers, and also solid-state lasers, for example Nd:YAG lasers. Corresponding measures for generating anchoring structures on thread surfaces are sufficiently familiar to a person skilled in the art, such that further details are not needed here.

In an alternative embodiment, the anchoring structures are formed integrally on the main body of the thread in such a way that they protrude permanently from the surface of the main body of the thread, i.e. cannot be converted to a position flush with the surface of the main body of the thread. Corresponding anchoring structures can be formed on the surface of the main body of the thread by injection moulding for example. In particular, the thread according to the invention can be produced by means of injection moulding.

The anchoring structures are hook-shaped, in particular barb-shaped, escutcheon-shaped, shield-shaped, scale-shaped, wedge-shaped, thorn-shaped, arrow-shaped, V-shaped and/or W-shaped, on the surface of the main body of the thread. The anchoring structures are particularly preferably barb-shaped or designed in the manner of barbs or designed as barbs on the surface of the main body of the thread. At their ends protruding away from the surface of the main body of the thread, the anchoring structures can also be sharp or pointed in order to facilitate penetration into tissues.

The anchoring structures can in principle be formed in different arrangements on the surface of the main body of the thread. For example, the anchoring structures can have a linear arrangement, a row by row arrangement, an offset arrangement, a zigzag arrangement, a spiral-shaped arrangement, a helical-shaped arrangement, a random arrangement, or combinations of these, in the longitudinal and/or transverse direction, preferably in the longitudinal direction, of the thread. The abovementioned arrangements are generally visible to the user only after removal of the coating. An arrangement is particularly preferred in which the anchoring structures are distributed across the entire surface of the main body of the thread, since in this case, after removal of the coating, the thread can anchor itself particularly firmly in a surrounding tissue area.

In another embodiment, the thread according to the invention has at least one set, in particular two, three or more sets, of anchoring structures on the surface of the main body of the thread. A set of anchoring structures is to be understood, within the meaning of the present invention, as an arrangement of anchoring structures, on the surface of the main body of the thread, that corresponds in respect of the configuration of the anchoring structures, in particular in respect of the height of the anchoring structures, the length of the anchoring structures, the cutting depth of the anchoring structures, the angle at which the anchoring structures protrude from the surface of the main body of the thread, the orientation of the anchoring structures and/or the shape of the anchoring structures. The number of sets and the configuration of their anchoring structures are also usually recognizable only after removal of the coating.
According to the invention, the anchoring structures can be formed unidirectionally on the surface of the main body of the thread. Preferably, the anchoring structures are formed bidirectionally on the surface of the main body of the thread. A bidirectional arrangement of anchoring structures is to be understood here as an arrangement in which the anchoring structures are formed in two different directions (bidirectionally) on the surface of the main body of the thread. Preferably, in the longitudinal direction of the thread, the anchoring structures for a first thread portion are formed in the direction of another, second thread portion, and the anchoring structures for the other, second thread portion are formed in the direction of the first thread portion. For example, seen in the longitudinal direction of the thread, the anchoring structures for a first thread portion can be oriented in the direction of the centre of the thread and, for another, second thread portion, are likewise oriented in the direction of the centre of the thread. The length of the thread portions can correspond to approximately half the thread length, such that the thread centre forms a kind of centre of symmetry.

In a particularly advantageous embodiment, the surface of the main body of the thread has at least two bidirectional arrangements of anchoring structures. In this case, it is preferable if, in relation to a first bidirectional arrangement of anchoring structures, a second bidirectional arrangement of anchoring structures is formed on the surface of the main body of the thread at approximately 180 degrees in the circumferential direction and preferably offset in relation to the first bidirectional arrangement. It is also possible, according to the invention, for the thread to have a total of three bidirectional arrangements of anchoring structures. In this case, it is preferable if, in relation to a first bidirectional arrangement of anchoring structures, a second bidirectional arrangement of anchoring structures is formed on the surface of the main body of the thread at approximately 120 degrees in the circumferential direction and preferably offset in relation to the first bidirectional arrangement, which second bidirectional arrangement of anchoring structures is in turn formed at approximately 120 degrees in the circumferential direction of the thread and preferably offset in relation to a third bidirectional arrangement of anchoring structures, such that the third bidirectional arrangement of anchoring structures is likewise formed at approximately 120 degrees in the circumferential direction of the thread and preferably offset in relation to the first bidirectional arrangement of anchoring structures.

In a further embodiment, the anchoring structures have a periodically changing, preferably alternating, orientation on the surface of the main body of the thread. For example, seen in the longitudinal direction of the thread, the anchoring structures for a first thread portion can be oriented in the direction of a second thread portion and, for the second thread portion, in the direction of the first thread portion, and for a third thread portion adjoining the second thread portion can be formed in the direction of a fourth thread portion adjoining the third thread portion and, for the fourth thread portion, can be formed in the direction of the third thread portion, etc.

In one possible embodiment, the coating of the thread can soften or melt in or upon contact with body fluids. It is particularly advantageous if the coating is converted to a low-viscosity melt. This has the advantage that the coating, after it has melted, can diffuse away from the surface of the main body of the thread relatively quickly and/or can be flushed away, as a result of which the anchoring structures on the surface of the main body of the thread are exposed.

The coating generally comprises a biocompatible material, in particular a biocompatible oligomer and/or polymer. The coating can in particular be composed mainly of the coating material. The coating material can be a synthetic polymer and/or naturally occurring polymer or biopolymer. Moreover, the polymer can be a copolymer. A copolymer is to be understood, within the meaning of the present invention, as a polymer composed of two or more monomer units. Accordingly, copolymers that can be used according to the invention can also be terpolymers or tetrapolymers. The polymer can in particular be a random copolymer or a block copolymer. According to the invention, the polymer can also be a block terpolymer. Polymers with a glass transition temperature of between 30 and 37°C are preferred.

The coating material is preferably chosen from the group consisting of proteins, polysaccharides, polyhydroxyalkanoates, oligohydroxyalkanoates, salts thereof, derivatives thereof, and combinations thereof. The polysaccharides can be chosen, for example, from the group comprising alkyl celluloses, hydroxyalkyl celluloses, carboxyalkyl celluloses, glycosaminoglycans, salts thereof, derivatives thereof, and combinations thereof. In a preferred embodiment, the coating material is chosen from the group consisting of polyvinyl alcohol (PVA), polyvinylpyrrolidone, collagen, gelatin, elastin, reticulin, albumin, dextran, amylose, amylose pectin, starch, chitosan, methyl cellulose, carboxymethyl cellulose, hydroxymethyl cellulose, hydroxyethyl cellulose, hydroxyethyl methyl cellulose, hydroxypropyl cellulose, hydroxypropyl methyl cellulose, hydroxybutyl cellulose, hyaluronic acid, heparin, heparin sulphate chondroitin-4 sulphate, chondroitin-6 sulphate, dermatan sulphate, keratan sulphate, salts thereof, derivatives thereof, and combinations thereof.

In a further embodiment, the abovementioned polyhydroxyalkanoates are chosen from the group consisting of polylactide, polyglycolide, polytrimethylene carbonate, polycaprolactone, poly-p-dioxanone, poly-3-hydroxybutyrate, poly-4-hydroxybutyrate, copolymers thereof, and mixtures thereof. The oligohydroxyalkanoates cited in the preceding paragraph can include lactide, glycolide, trimethylene carbonate, caprolactone and/or p-dioxanone.

The main body of the thread and the anchoring structures can in principle be formed from all the materials suitable for this purpose, preferably polymers. The main body of the thread and the anchoring structures can comprise different materials, in particular different polymers, or can be formed from different materials, in particular different polymers. The main body of the thread and the anchoring structures preferably comprise the same materials, in particular the same polymers, or are formed from the same materials, in particular the same polymers. The polymers can be resorbable, partially resorbable or non-resorbable polymers. The polymers can be present as homopolymers, copolymers, terpolymers or tetrapolymers. The polymers can also be block polymers, in particular block copolymers or block terpolymers. The use of random or alternating copolymers or terpolymers is possible according to the invention.

If the main body of the thread and/or the anchoring structures are formed from resorbable polymers, the polymers are preferably chosen from the group including polylactide, polyglycolide, poly-ε-caprolactone, poly-para-dioxanone, polytrimethylene carbonate, polyhydroxybutyrate, copolymers thereof, terpolymers thereof and mixtures thereof. A suitable polyhydroxybutyrate is poly-3-hydroxybutyrate and/or poly-4-hydroxybutyrate. Resorbable copolymers or terpolymers are particularly preferred, in particular resorbable block copolymers or block terpolymers which comprise a monomer from the group including lactide, glycolide, trimethylene carbonate, para-dioxanone, ε-caprolactone, 3-hydroxybutyrate, 4-hydroxybutyrate, and combinations thereof. For example, the main body of the thread and/or the anchoring structures can be made from a triblock terpolymer, comprising glycolide, trimethylene carbonate and ε-caprolactone. A triblock terpolymer of this kind is commercially available under the name Monosyn®.

The non-resorbable materials used for the main body of the thread and/or the anchoring structures can be polymers from the group including polyolefins, polyesters, polyamides, polyurethanes, copolymers thereof, terpolymers thereof, and mixtures thereof. Polypropylene, polyethylene terephthalate, polypropylene terephthalate, polybutylene terephthalate, polytetrafluoroethylene, polyvinylidendifluoride, polytetrafluoropropylene, polyhexafluoropropylene, linear and preferably aliphatic polyurethanes and/or nylon are mentioned by way of example.

According to a further embodiment, the thread according to the invention can contain active substances, in particular antimicrobial, disinfecting, anti-inflammatory, analgesic, growth-promoting and/or deodorizing active substances. The active substances can be contained in the main body of the thread, in the anchoring structures and/or in the coating. However, the active substances are generally contained in the main body of the thread and/or in the anchoring structures, since in this case they are able to act for longer in the body of a human or animal patient.

In a further embodiment, the thread, in particular the main body, is a mass or solid thread, in particular a mass or solid main body. This preferably means that the thread, in particular the main body, has no lumen.

According to another embodiment, the thread is designed as a hollow thread, in particular as a tubular thread, preferably as a tube or hose. Preferably, the hollow thread comprises a closed wall, wherein the ends of the thread are preferably open. Such a hollow thread may be produced, by way of example, by means of extrusion. The anchoring structures may be designed as cuts into the wall of the hollow thread, wherein the cuts preferably do not break through the wall of the hollow thread. As an alternative or in combination, the anchoring structures may be designed as breakthroughs, i.e. the anchoring structures may be formed completely breaking through the wall of the hollow thread. Hollow threads according to the present invention may be used as self-anchoring infusion tubes, delivery tubes, catheters, distribution systems for medical agents, in particular liquid medical agents, drug-release-systems and/or drainage systems, in particular drainage tubes.

More specifically, the main body may be present as a hollow main body, in particular a tubular main body, preferably as a tube or hose. With respect to further details and advantages, reference is made in its entirety to the previous description.

The thread according to the invention, in particular the main body of the thread, generally has a circular cross section. However, other cross-sectional shapes are also conceivable in principle. For example, the thread, in particular the main body of the thread, can have an oval, triangular, triple-lobed, square, trapezoidal, rhomboid, pentagonal, hexagonal, star-shaped or cross-shaped cross section.

The thread according to the invention can in principle be a monofilament, a pseudomonofilament or multifilament. If the thread is a multifilament, in particular multifilament yarn, the thread can additionally be present in a braided form. According to the invention, however, it is particularly preferable if the thread is a monofilament.

The thread is a knotless or self-fixing surgical suture material.

In another embodiment, the thread is armed at least at one end, in particular at both ends, with a surgical insertion instrument, generally a surgical needle. It is particularly advantageous if the surgical insertion instrument for arming the thread has a hole into which the thread can be introduced. After the thread has been introduced into the hole, the surgical insertion instrument can then be squeezed together or crimped in the area of the bore.

The thread according to the invention is generally present in a stretched form. This is especially the case when the thread is designed as surgical suture material. In principle, however, it is also possible for the thread according to the invention to be present in an unstretched form.

In a further embodiment, the thread is in a sterilized and ready-to-use state and, in particular, packaged.

A further aspect of the present invention concerns a surgical implant that comprises at least one thread according to the present invention. The implant is preferably formed as a textile implant. Examples of possible implants are chosen from the group including hernia meshes, prolapse meshes, urinary incontinence bands and wound dressings. However, the surgical implant is particularly preferably formed as surgical suture material comprising at least one thread according to the present invention. For further features and details, particularly regarding the thread according to the invention, reference is made in full to the preceding description.

The present invention also relates to a surgical kit. The kit comprises at least one surgical insertion instrument, in particular a surgical needle or a surgical cannula, and at least one thread according to the present invention. For further features and details, reference is again made in full to the preceding description.

The invention further relates to a method for producing a thread according to the invention. To produce the thread, a coating that can be removed by means of liquids, in particular body fluids, is applied to the surface of a main body of the thread, on the surface of which main body anchoring structures are formed for anchoring in biological tissues, in particular human and/or animal tissues.

In a preferred embodiment, the coating is applied to the surface of the main body of the thread by means of sheathing extrusion, as a result of which at least some of the anchoring structures, preferably all of them, are covered, in particular embedded. The technique of sheathing extrusion of threads is sufficiently familiar to a person skilled in the art, and no further details are therefore given at this point.

In an alternative embodiment, the coating is applied to the surface of the main body of the thread by means of the following steps:
a) immersing the main body of the thread in a solution or suspension that contains a coating material,
b) removing the main body of the thread from the solution or suspension,
c) drying the thread body in order to form the thread.

Water, methanol, ethanol, propanol, isopropanol and/or acetone are suitable in principle as solvents for preparing a solution or suspension containing the coating material. The coating material can be present in the solution or suspension at a concentration of between 5 and 70% by weight, in particular 10 and 50% by weight, relative to the total weight of the solution or suspension. According to the invention, provision can in particular be made for the coating material to precipitate during immersion of the main body of the thread into the solution or suspension, as a result of which a deposit forms on the main body of the thread to be coated, which deposit preferably completely covers the main body of the thread. The precipitation of the coating material can be brought about, for example, by a change of the concentration, temperature and/or pH value of the solution or suspension. If appropriate, step a) of the method according to the invention can also be repeated several times, in order in this way to achieve, for example, a complete coating of the main body of the thread and/or a greater layer thickness.

To apply the coating, the main body of the thread according to another embodiment can be pulled through a tubular auxiliary instrument, the diameter of which corresponds to the diameter of the main body of the thread without anchoring structures, and, as the main body of the thread emerges from the auxiliary instrument, the coating is applied directly to the surface of the main body of the thread. If the main body of the thread is pulled through an auxiliary instrument of this kind, the anchoring structures (protruding from the main body of the thread) are pressed onto the surface of the main body of the thread. The anchoring structures can be fixed or held in this position by the applied coating. According to the invention, it is also possible in principle for the coating material to be injected through nozzles into the auxiliary instrument.

In a further embodiment, the main body of the thread, before being immersed in a solution or suspension containing a suitable coating material, is introduced into a tubular auxiliary instrument which is provided with openings and of which the diameter corresponds to the diameter of the main body of the thread without anchoring structures. In this embodiment, the auxiliary instrument (including the inserted main body of the thread) is then immersed in the solution or suspension.

The thread can be dried in principle at room temperature, in a heating oven, by means of a hot current of air, by means of infrared radiation or other techniques known to a person skilled in the art.

A preferred area of use, not forming part of the invention, of the thread according to the invention is plastic surgery. There, the thread is preferably used for tightening the skin or for lifting, for example for eyebrow lifts. Other areas of use, not forming part of the invention, are for correction of the cheek and/or chin lines. In addition, the thread according to the invention is also suitable for other surgical indications, not forming part of the invention, in particular for indications in which the use of conventional suture materials is difficult on account of steric hindrance. For example, the thread according to the invention can be used in laparoscopic interventions, particularly for fixing meshes, for example hernia meshes, prolapse meshes or urinary incontinence meshes.

Accordingly, a further aspect of the disclosure concerns the not forming part of the invention use of the thread for producing a fixing means for implants, in particular textile implants, preferably meshes. A further possible area of use, not forming part of the invention, of the thread is in the formation of anastomoses, in particular vascular or intestinal anastomoses. A further area of use, not forming part of the invention, of the thread according to the invention concerns the production of fixing means for implants, in particular textile implants, preferably meshes. For further features and details, reference is made to the preceding description.

By means of the present invention, a surgical thread is made available which is suitable especially for use as a knotless or self-fixing surgical suture material. The main body of the thread has a coating that can be removed by means of liquids, preferably body fluids, and that preferably covers, in particular embeds, all of the anchoring structures formed on the surface of the main body of the thread. The outer surface of the thread is preferably made smooth as a result of the coating. In this way, the thread can be pulled gently and with the least possible trauma into a biological tissue, because the anchoring structures do not come into contact with the tissue during this procedure. It is therefore not necessary to use insertion devices as described in the introduction. This allows a physician or surgeon to manoeuvre the thread in a manner largely free of complications and risks.

Moreover, by virtue of the coating provided according to the invention, a physician or surgeon also has more time to position the thread correctly from the medical or surgical point of view. If appropriate, the thread can also be repositioned several times, without this resulting in additional tissue trauma. It is particularly advantageous if the anchoring structures are designed elastically or have a certain elasticity, such that they right themselves preferably independently after removal of the coating.

In the schematic drawings:
Figure 1 shows an embodiment of the thread according to the invention,
Figure 2 shows another embodiment of the thread according to the invention.

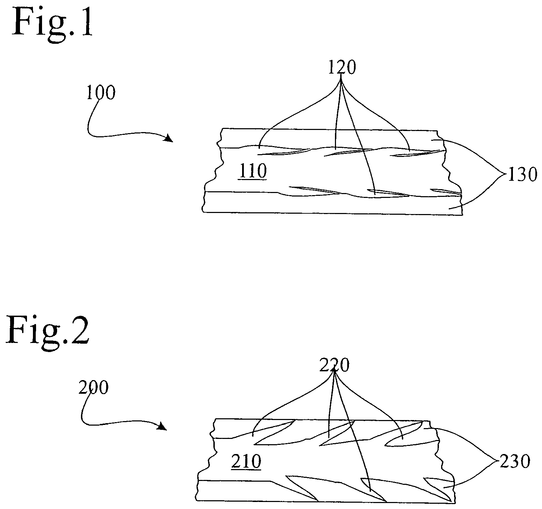

The thread 100 shown schematically in Figure 1 has a main body 110, and anchoring structures 120 formed on the surface of the main body 110 of the thread. The main body 110 of the thread is covered preferably completely by a coating 130. The anchoring structures 120 are fixed, by the coating 130, in a position in which they bear as tightly as possible on the surface of the main body of the thread. The anchoring structures 120 can be formed, for example, as cuts on the surface of the main body 110 of the thread. The coating 130 has the purpose of ensuring that the thread can be pulled into a biological tissue with the least possible trauma and can be pulled through said biological tissue with the least possible trauma. The coating 130 can preferably dissolve in or upon contact with body fluids. Ideally, however, the coating material dissolves only after a delay, such that the thread 100 can, if necessary, be repositioned by a physician or surgeon and can, if appropriate, also be repositioned several times. Alternatively or in combination with this, the coating 130 can also be removed by means of flushing liquids suitable for this purpose. Suitable flushing liquids are in principle all aqueous biocompatible liquids. Examples that may be mentioned are physiological buffer solutions, electrolyte solutions, salt solutions or sugar solutions. The flushing liquids can, if appropriate, contain active substances, for example antimicrobial, disinfecting and/or anti-inflammatory active substances.

The thread 200 according to the invention shown schematically in Figure 2 likewise has a main body 210, and anchoring structures 220 on the surface of the main body 210 of the thread. The anchoring structures 220 are covered by a coating 230 of the main body 210 of the thread in such a way that the anchoring structures 220 are embedded in the coating 230 and are fixed, by the coating 230, in a position protruding from the surface of the main body of the thread. For further features and details, reference is made to the description of Figure 1.

### Examples

### 1.1 Production of a barbed thread made of poly-para-dioxanone

An undrawn monofile spinning thread made of poly-para-dioxanone was produced by means of thermoplastic extrusion. The monofile thread had a diameter of 0,95 mm. Afterwards, 50 cuts were made into the undrawn spinning thread by means of a cutting apparatus.

The cuts were made under a cutting angle of 20°, in a cutting depth of 31 % in relation to the thread's diameter and in a distance to each other of 0,28 mm. The monofile thread was rotated around its longitudinal axis under 120° after each cut. Thus, a helix-shaped arrangement of cuts was formed on the thread. The cut-in thread was drawn to the fourfold of its original length in a slit heater at a temperature of 70°C. Thus, protruding barbs were formed. The self-anchoring monofile thread had a linear tensile strength of 35,8 N at an elongation of break of 29 % and a diameter of 0,49 mm in thread areas where no barbs were present. The heads of the protruding barbs protruded about 0,27 mm from the main thread body.

The thread was attached at one end to a G21-cannula (without luer-lock) in order to investigate the pull-out strength in relation to animal tissue. The thread was attached to the G21-cannula in such a way that the barbs were orientated away from the needle. Thus, it was ensured that the barbs did not block when the thread was pulled into the animal tissue (swine abdomen with a callosity, thickness about 2 cm). The pull-through strength in the slip direction amounted to 1,1 N. After pulling the thread into the tissue, a pull-out strength of 8,4 N was measured by means of a tensile testing machine.

### 1.2 Production of a tube made of polyvinylalcohol (PVA)

A tube was extruded from a thermoplastic processable PVA (type Mowiflex TC, company Kuraray). The extrusion was performed by means of a Haake twin-screw extruder (TW100), a catheter nozzle (ring diameter 3 mm, support air nozzle 1,0 mm) without spinning pump using a screw rotational frequency of 10 U/min and a haul-off speed of 5 m/min under support air (nitrogen, 15 scale parts) at a nozzle temperature of 188°C. The extruded tube was spooled on a drum winder in a single-layer. The tube had a lumen diameter of 1,01 mm and a wall thickness of 0,18 mm.

### 1.3 Drawing of the tube produced according to Example 1.2 on the drawn barbed thread as produced according to Example 1.1

The undrawn PVA-tube according to Example 1.2 was cut to a length of 15 cm. The thread was pulled into the cut tube, wherein thread areas having no barbs were pulled into the tube at first. Further, the thread was pulled into the cut tube in such a way that the barbs were orientated in the opposing direction. Afterwards, the thread's portion having barbs were placed within the tube in such a way that said portion protruded into the lumen of the tube over a length of 5 cm starting from one tube end. At this end, the tube together with the thread was fixed in a clamp. Subsequently, the tube (nearly the tube) having the barbed thread within its lumen was drawn at maximum in a heating channel at a temperature of 80°C. Due to the reduction of the lumen diameter and the wall thickness, a sheath having a thickness of 0,09 mm was formed on the barbed thread. Thus, the barbs were pressed on the surface of the thread and at the same time encased by the sheath. The tube could not be mechanically separated from the thread. The surface of the tube-thread-construct was slightly corrugated. In this situation, the barbs did not show any anchoring function.

Afterwards, the PVA-sheath was cut over a length of 2 mm away from the thread body, in order to attach the thread body to a G21-cannula.

When pulling through a swine abdomen with callosity, it turned out that the pull-through strength in slip direction was reduced to 0,3 N, due to the sheath. Further, a pull-out strength in blocking direction of the barbs amounted to 0,7 N. This advantageously offers the possibility to reposition the thread within the tissue, if appropriate, as long as the sheath is intact. The experiment was repeated after the meat was stored in an aqueous medium for a time of 5 min. The corresponding pull-out strength amounted to 5,6 N. After further 5 min, the pull-out strength amounted to 6,9 N which was close to the pull-out strength of the thread without sheath, which means that the sheath was basically dissolved.

### 1.4 Common drawing of the cut-in thread according to Example 1.1 and the PVA tube according to Example 1.2

The cut-in, yet undrawn thread according to Example 1.1 was pulled into the undrawn tube according to Example 1.2. After cutting to a length of 10 cm, the tube and thread commonly were drawn to maximal length in a heating channel at a temperature of 80°C. The result was basically the same as obtained in Example 1.3. Further, the pull-through strength and pull-out strength were basically identical to the corresponding results of Example 1.3. Furthermore, when immersing the tube-thread-construct in water at a temperature of 22°C, it turned out that the tube was basically dissolved after 4 min.

cal to the corresponding results of Example 1.3. Furthermore, when immersing the tube-thread-construct in water at a temperature of 22°C, it turned out that the tube was basically dissolved after 4 min.

## Claims

1. A knotless or self-fixing surgical suture material thread (100; 200) comprising a main body (110; 210), and anchoring structures (120; 220) that are formed on the surface of the main body (110; 210) of the thread and are used for anchoring in biological tissues, wherein at least some of the anchoring structures (120; 220) are covered at least partially by a coating (130; 230) of the main body (110; 210) of the thread, which coating (130; 230) can be removed by means of liquids, wherein at least some of the anchoring structures (120; 220) are completely embedded in the coating (130; 230) and the anchoring structures (120; 220) are fixed by the coating, in a certain position on the surface of the main body (110; 210) of the thread (100;200), **characterized in that** the anchoring structures (120; 220) are hook-shaped, escutcheon-shaped, shield-shaped, scale-shaped, wedge-shaped, thorn-shaped, arrow-shaped, V-shaped and/or W-shaped on the surface of the main body (110; 210) of the thread (100; 200).

2. Thread (100; 200) according to claim 1, **characterized in that** at least some of the anchoring structures (120; 220) are fixed, by the coating (130; 230), in a position protruding from the surface of the main body (110; 210) of the thread.

3. Thread (100; 200) according to claim 1, **characterized in that** at least some of the anchoring structures (120; 220) are fixed, by the coating (130; 230), in a position bearing on the surface of the main body (110; 210) of the thread.

4. Thread (100; 200) according to one of the preceding claims, **characterized in that** the coating (130; 230) is a coating that can dissolve in liquids, in particular body fluids.

5. Thread (100; 200) according to one of the preceding claims, **characterized in that** the coating (130; 230) is formed in the manner of a sheath on the surface of the main body (110; 210) of the thread, which sheath preferably completely surrounds the main body (110; 210) of the thread.

6. Thread (100; 200) according to one of the preceding claims, **characterized in that**, by means of the coating (130; 230), the thread (100; 200) has a friction-reducing outer surface, preferably substantially flat outer surface.

7. Thread (100; 200) according to one of the preceding claims, **characterized in that** the coating (130; 230) is porous, preferably having a porosity of between 30 and 90%, in particular 60 and 80%, relative to the total volume of the coating (130; 230).

8. Thread (100; 200) according to one of the preceding claims, **characterized in that** the anchoring structures (120; 220) are formed as cuts in the surface of the main body (110; 210) of the thread.

9. Thread (100; 200) according to one of the preceding claims, **characterized in that** the anchoring structures (120; 220) are barb-shaped or designed in the manner of barbs on the surface of the main body (110; 210) of the thread.

10. Thread (100; 200) according to one of the preceding claims, **characterized in that** the anchoring structures (120; 220) on the surface of the main body of the thread have a periodically changing, preferably alternating, orientation.

11. Thread (100; 200) according to one of the preceding claims, **characterized in that** the coating (130; 230) can soften upon contact with body fluids, preferably forming a low-viscosity melt.

12. Thread (100; 200) according to one of the preceding claims, **characterized in that** the coating (130; 230) comprises a biocompatible material, in particular an oligomer and/or polymer, preferably chosen from the group consisting of proteins, polysaccharides, polyhydroxyalkanoates, oligohydroxyalkanoates, derivatives thereof, and combinations thereof.

13. Thread (100; 200) according to claim 12, **characterized in that** the material is chosen from the group consisting of polyvinyl alcohol (PVA), polyvinylpyrrolidone, collagen, gelatin, elastin, albumin, dextran, amylose, amylose pectin, starch, chitosan, carboxymethyl cellulose, hydroxyethyl cellulose, hyaluronic acid, chondroitin sulphate, derivatives thereof, and combinations thereof.

14. Surgical implant, preferably in the form of a surgical suture material, comprising at least one thread (100; 200) according to one of the preceding claims.

15. Surgical kit, comprising at least one surgical insertion instrument and at least one thread (100; 200) according to one of claims 1 to 13 or a surgical implant according to Claim 14.

16. Method for producing a thread (100; 200) according to one of claims 1 to 13, **characterized in that** a coating (130; 230) that can be removed by means of liquids is applied to the surface of a main body (110; 210) of the thread, on the surface of which main body (110; 210) anchoring structures (120; 220) are formed for anchoring in biological tissues.

## Patentansprüche

1. Knotenloses oder selbstfixierendes chirurgisches Nahtmaterial als Faden (100; 200), umfassend einen Grundkörper (110; 210) und Verankerungsstrukturen (120; 220), die auf der Oberfläche des Grundkörpers (110; 210) des Fadens ausgebildet sind und zur Verankerung in biologischen Geweben verwendet werden, wobei zumindest ein Teil der Verankerungsstrukturen (120; 220) zumindest teilweise von einer mittels Flüssigkeiten entfernbaren Beschichtung (130; 230) des Grundkörpers (110; 210) des Fadens bedeckt ist, wobei zumindest eine Teil der Verankerungsstrukturen (120; 220) in der Beschichtung (130; 230) vollständig eingebettet vorliegt und die Verankerungsstrukturen (120; 220) durch die Beschichtung in einer bestimmten Position auf der Oberfläche des Grundkörpers (110; 210) des Fadens (100; 200) fixiert sind,
**dadurch gekennzeichnet, dass**
die Verankerungsstrukturen (120; 220) haken-, wappen-, schild-, schuppen-, keil-, stachel-, pfeil-, V- und/oder W-förmig auf der Oberfläche des Grundkörpers (110; 210) des Fadens (100; 200) ausgebildet sind.

2. Faden (100; 200) nach Anspruch 1, **dadurch gekennzeichnet, dass** zumindest ein Teil der Verankerungsstrukturen (120; 220) durch die Beschichtung (130; 230) in einer von der Oberfläche des Fadengrundkörpers (110; 210) abstehenden Position fixiert ist.

3. Faden (100; 200) nach Anspruch 1, **dadurch gekennzeichnet, dass** zumindest ein Teil der Verankerungsstrukturen (120; 220) durch die Beschichtung (130; 230) in einer auf der Oberfläche des Fadengrundkörpers (110; 210) anliegenden Position fixiert ist.

4. Faden (100; 200) nach einem der vorhergehenden Ansprüche, **dadurch gekennzeichnet, dass** es sich bei der Beschichtung (130; 230) um eine in Flüssigkeiten, insbesondere Körperflüssigkeiten, auflösbare Beschichtung handelt.

5. Faden (100; 200) nach einem der vorhergehenden Ansprüche, **dadurch gekennzeichnet, dass** die Beschichtung (130; 230) nach Art einer Ummantelung auf der Oberfläche des Fadengrundkörpers (110; 210) ausgebildet ist, welche den Fadengrundkörper (110; 210) vorzugsweise vollflächig umgibt.

6. Faden (100; 200) nach einem der vorhergehenden Ansprüche, **dadurch gekennzeichnet, dass** der Faden (100; 200) durch die Beschichtung (130; 230) eine reibungsmindernde, vorzugsweise eine im Wesentlichen glatte, Außenoberfläche besitzt.

7. Faden (100; 200) nach einem der vorhergehenden Ansprüche, **dadurch gekennzeichnet, dass** die Beschichtung (130; 230) porös ausgebildet ist, vorzugsweise eine Porosität zwischen 30 und 90 %, insbesondere 60 und 80 %, aufweist, bezogen auf das Gesamtvolumen der Beschichtung (130; 230).

8. Faden (100; 200) nach einem der vorhergehenden Ansprüche, **dadurch gekennzeichnet, dass** die Verankerungsstrukturen (120; 220) als Einschnitte in die Oberfläche des Fadengrundkörpers (110; 210) ausgebildet sind.

9. Faden (100; 200) nach einem der vorhergehenden Ansprüche, **dadurch gekennzeichnet, dass** die Verankerungsstrukturen (120; 220) widerhakenförmig oder nach Art von Widerhaken auf der Oberfläche des Fadengrundkörpers (110; 210) ausgebildet sind.

10. Faden (100; 200) nach einem der vorhergehenden Ansprüche, **dadurch gekennzeichnet, dass** die Verankerungsstrukturen (120; 220) auf der Fadengrundkörperoberfläche eine sich periodisch ändernde, vorzugsweise alternierende, Orientierung besitzen.

11. Faden (100; 200) nach einem der vorhergehenden Ansprüche, **dadurch gekennzeichnet, dass** die Beschichtung (130; 230) bei Kontakt mit Körperflüssigkeiten erweichbar ist, vorzugsweise unter Ausbildung einer niederviskosen Schmelze.

12. Faden (100; 200) nach einem der vorhergehenden Ansprüche, **dadurch gekennzeichnet, dass** die Beschichtung (130; 230) ein bioverträgliches Material, insbesondere ein Oligomer und/oder Polymer, vorzugsweise ausgewählt aus der Gruppe bestehend aus Proteine, Polysaccharide, Polyhydroxyalkanoate, Oligohydroxyalkanoate, Derivate davon und Kombinationen davon, aufweist.

13. Faden (100; 200) nach Anspruch 12, **dadurch gekennzeichnet, dass** das Material aus der Gruppe bestehend aus Polyvinylalkohol (PVA), Polyvinylpyrrolidon, Kollagen, Gelatine, Elastin, Albumin, Dextran, Amylose, Amylospektin, Stärke, Chitosan, Carboxymethylcellulose, Hydroxyethylcellulose, Hyaluronsäure, Chondroitinsulfat, Derivate davon und Kombinationen davon ausgewählt ist.

14. Chirurgisches Implantat, vorzugsweise in Form eines chirurgischen Nahtmaterials, umfassend zumindest einen Faden (100; 200) nach einem der vorhergehenden Ansprüche.

15. Chirurgisches Kit, umfassend zumindest ein chirurgisches Einführinstrument und zumindest einen Faden (100; 200) nach einem der Ansprüche 1 bis 13 oder ein chirurgisches Implantat nach Anspruch 14.

16. Verfahren zur Herstellung eines Fadens (100; 200) nach einem der Ansprüche 1 bis 13, **dadurch gekennzeichnet, dass** eine mittels Flüssigkeiten entfernbare Beschichtung (130; 230) auf die Oberfläche eines Fadengrundkörpers (110; 210), auf dessen Oberfläche Verankerungsstrukturen (120; 220) zur Verankerung in biologischen Geweben ausgebildet sind, aufgebracht wird.

## Revendications

1. Fil en matériau de suture chirurgicale sans nœud ou autofixant (100 ; 200) comprenant un corps principal (110 ; 210), et des structures d'ancrage (120 ; 220) qui sont formées à la surface du corps principal (110 ; 210) du fil et qui sont utilisées pour un ancrage dans des tissus biologiques, dans lequel au moins certaines des structures d'ancrage (120 ; 220) sont couvertes au moins partiellement par un revêtement (130 ; 230) du corps principal (110 ; 210) du fil, lequel revêtement (130 ; 230) peut être éliminé au moyen de liquides, dans lequel au moins certaines des structures d'ancrage (120 ; 220) sont entièrement intégrées dans le revêtement (130 ; 230) et les structures d'ancrage (120 ; 220) sont fixées par le revêtement, dans une certaine position à la surface du corps principal (110 ; 210) du fil (100 ; 200), **caractérisé en ce que** les structures d'ancrage (120 ; 220) sont en forme de crochet, en forme d'écusson, en forme de bouclier, en forme d'écaille, en forme de coin, en forme d'épine, en forme de flèche, en forme de V et/ou en forme de W à la surface du corps principal (110 ; 210) du fil (100 ; 200).

2. Fil (100 ; 200) selon la revendication 1, **caractérisé en ce qu'**au moins certaines des structures d'ancrage (120 ; 220) sont fixées, par le revêtement (130 ; 230), dans une position dépassant de la surface du corps principal (110 ; 210) du fil.

3. Fil (100 ; 200) selon la revendication 1, **caractérisé en ce qu'**au moins certaines des structures d'ancrage (120 ; 220) sont fixées, par le revêtement (130 ; 230), dans une position portante à la surface du corps principal (110 ; 210) du fil.

4. Fil (100 ; 200) selon l'une des revendications précédentes, **caractérisé en ce que** le revêtement (130 ; 230) est un revêtement qui peut se dissoudre dans des liquides, en particulier des fluides corporels.

5. Fil (100 ; 200) selon l'une des revendications précédentes, **caractérisé en ce que** le revêtement (130 ; 230) est formé à la manière d'une gaine sur la surface du corps principal (110 ; 210) du fil, laquelle gaine entoure de préférence entièrement le corps principal (110 ; 210) du fil.

6. Fil (100 ; 200) selon l'une des revendications précédentes, **caractérisé en ce que**, au moyen du revêtement (130 ; 230), le fil (100 ; 200) possède une surface externe réduisant la friction, de préférence une surface externe sensiblement plate.

7. Fil (100 ; 200) selon l'une des revendications précédentes, **caractérisé en ce que** le revêtement (130 ; 230) est poreux, ayant de préférence une porosité entre 30 et 90 %, en particulier 60 et 80 %, par rapport au volume total du revêtement (130 ; 230).

8. Fil (100 ; 200) selon l'une des revendications précédentes, **caractérisé en ce que** les structures d'ancrage (120 ; 220) sont formées en tant que découpes dans la surface du corps principal (110 ; 210) du fil.

9. Fil (100 ; 200) selon l'une des revendications précédentes, **caractérisé en ce que** les structures d'ancrage (120 ; 220) ont la forme de barbes ou sont conçues à la manière de barbes sur la surface du corps principal (110 ; 210) du fil.

10. Fil (100 ; 200) selon l'une des revendications précédentes, **caractérisé en ce que** les structures d'ancrage (120 ; 220) à la surface du corps principal du fil ont une orientation changeant périodiquement, de préférence alternée.

11. Fil (100 ; 200) selon l'une des revendications précédentes, **caractérisé en ce que** le revêtement (130 ; 230) peut s'amollir lors d'un contact avec des fluides corporels, de préférence en formant une fonte à faible viscosité.

12. Fil (100 ; 200) selon l'une des revendications précédentes, **caractérisé en ce que** le revêtement (130 ; 230) comprend un matériau biocompatible, en particulier un oligomère et/ou un polymère, de préférence choisi dans le groupe constitué par des protéines, des polysaccharides, des polyhydroxyalkanoates, des oligohydroxyalkanoates, des dérivés de ceux-ci, et des combinaisons de ceux-ci.

13. Fil (100 ; 200) selon la revendication 12, **caractérisé en ce que** le matériau est choisi dans le groupe consistant en alcool polyvinylique (PVA), polyvinylpyrrolidone, collagène, gélatine, élastine, albumine, dextrane, amylose, pectine d'amylose, amidon, chitosane, carboxyméthylcellulose, hydroxyéthylcellulose, acide hyaluronique, sulfate de chondroïtine, des dérivés de ceux-ci et des combinaisons de ceux-ci.

14. Implant chirurgical, de préférence sous la forme d'un matériau de suture chirurgicale, comprenant au moins un fil (100 ; 200) selon l'une des revendications précédentes.

15. Kit chirurgical, comprenant au moins un instrument d'insertion chirurgical et au moins un fil (100 ; 200) selon l'une des revendications 1 à 13 ou un implant chirurgical selon la revendication 14.

16. Procédé de production d'un fil (100 ; 200) selon l'une des revendications 1 à 13, **caractérisé en ce qu'**un revêtement (130 ; 230) qui peut être éliminé au moyen de liquides est appliqué à la surface d'un corps principal (110 ; 210) du fil, à la surface duquel sont formées des structures d'ancrage (120 ; 220) du corps principal (110 ; 210) pour un ancrage dans des tissus biologiques.
